# EUROPEAN PATENT SPECIFICATION

(11) **EP 0 381 700 B1**
(45) Date of publication and mention of the grant of the patent: **24.05.1995**
(21) Application number: 88909661.6
(22) Date of filing: 12.10.1988
(51) Int. Cl.: A61K 31/70

(54) **The use of a fructooligosaccharid in the manufacture of a bacteriostatic composition for inhibiting the growth of Salmonella and a process for inhibiting the growth of Salmonella in a food animal**
VERWENDUNG VON EINEM FRUCTO-OLIGOSACCHARID ZUR HERSTELLUNG EINER BACTERIOSTATISCHEN ZUSAMMENSETZUNG ZUR VORBEUGUNG DES WACHSENS VON SALMONELLEN UND EIN VERFAHREN ZUR VORBEUGUNG DES WACHSENS VON SALMONELLEN
Utilisation d'un fructo-oligosaccharide dans la fabrication d'une composition bactériostatique pour inhiber la croissance des salmonelles et procédé pour inhiber la croissance des salmonelles chez un animal destiné à l'alimentation humaine

(30) Priority: 13.10.1987 US 107115
(43) Date of publication of application: 16.08.1990
(73) Proprietor: BEGHIN-MEIJI INDUSTRIES, F-75008 Paris (FR)
(72) Inventor: SPEIGHTS, Robert, M., Arvada, CO 80004 (US); PERNA, Peter, J., Boulder, CO 80303 (US)
(74) Representative: David, Daniel
(86) International application number: PCT/US88/03552
(87) International publication number: WO 89/03218

(56) References cited:
- GB-A- 2 105 338
- GB-A- 2 179 946
- GB-B- 2 072 679
- US-A- 3 728 132
- US-A- 4 024 251
- US-A- 4 335 107
- US-A- 4 435 389
- US-A- 4 581 227
- US-A- 4 681 771
- US-A- 4 689 226
- US-A- 4 726 948
- US-A- 4 734 402
- US-A- 4 762 822
- CHEMICAL ABSTRACTS, vol. 100, no. 17, 23rd April 1984, page 508, abstract no.137377f, Columbus, Ohio, US; & JP-A-58 201 980 (MEIJI SEIKA KAISHA, LTD) 25-11-1983
- THE VETERINARY RECORD, vol. 106, no. 3, 19th January 1980, page 61; E.M. BARNESet al.: "Competitive exclusion of salmonellas from the newly hatched chick"
- AVIAN DISEASES, vol. 29, no. 4, 1985, pages 1273-1276; O.M. WEINACK et al.:"Further studies on competitive exclusion of Salmonella typhimurium bylactobacilli in chickens"
- S. Stavric, "MICROBIAL COLONIZATION CONTROL OF CHICKEN INTESTINE USING DEFINED CULTURES" FOOD TECHNOLOGY 41 (7), July 1987, pp 93-98
- TSUNEYUKI OKU ET AL, "Nondigestibility of a new sweetner, "Neosugar", in the rat", Journal of nutrition, Vol. 114 No. 9, pp 1574-81 (1984)

## Description

### Field of the Invention

The present invention relates generally to the inhibition of growth of Salmonella. More specifically, the invention relates to a method for inhibiting the growth of Salmonella in the intestines of food animals to prevent Salmonella infections in humans.

### Background of the Invention

Salmonella bacteria are well known human pathogens. The most common means of human infection is by ingestion of contaminated foods. Many species of Salmonella are recognized as common microflora in the intestines of food animals, such as poultry and beef.

Various compositions are known for treatment of Salmonella poisoning in humans. For example, chloramphenicol, ampicillin, and trimethoprim-sulfa, are known to be effective against Salmonella organisms. While such compositions are generally useful against Salmonella, the ideal method for controlling Salmonella poisoning is prevention of infection. Proper cleaning of meat and dairy products and thorough cooking can prevent human infection by Salmonella.

One embodiment of the present invention is a method for controlling Salmonella poisoning in humans by inhibiting the growth of Salmonella populations in food animals. In this manner, fewer organisms are present in the food animals, and therefore, the chance of transmission to humans is smaller. This method involves introducing an effective compound for the inhibition of growth of Salmonella to the intestinal tract of food animals.

Specific embodiments of the effective compound of the present method are produced by Meiji Seika Kaisha, Ltd. under the trade name "Neosugar". For example, in Oku et al., Nondigestibility of a New Sweetener, "Neosugar," in the Rat, J. of Nutrition, v. 114, No. 9, pp. 1575-81 (1984), Neosugar is described as a mixture of 1-kestose, nystose, and 1-fructofuranosyl nystose which was studied for digestibility in rats. See also U.S. Patent US-A-4,681,771 to Adachi, et al. (July 21, 1987), U.K. Patent GB-B-2,072,679 and U.K. Patent GB-B-2,150,338, owned by Meiji Seika, which discuss the use of Neosugar compositions as low-cariogenic and low-calorie sweeteners.

Similar compounds are known for a variety of other uses. In European Patent Application EP-A-0188047, a process for preparing a compound termed "fructo-oligosaccharose" was disclosed. The process involves culturing an Aureo-bacidium species to produce the enzyme fructosyl-transferase. The culture medium is then contacted with sucrose to provide a substrate for the production of this fructo-oligosaccharose by the enzyme.

European Patent Application EP-A-0133547, describes an animal feed for preventing scours (diarrhea) which includes fructo-oligosaccharides produced by the action of fructosyl transferase on sucrose.

U.S. Patent US-A-4,496,550 to Lindahl, et al. (January 29, 1985) and U. S. Patent US-A-4,401,662 to Lormeau, et al. (August 30, 1983) discuss the use of mixtures of oligosaccharides to counteract or prevent coagulation of blood to revent arterial thrombosis.

U.S. Patent US-A-3,701,714 to Shigetaka (October 31, 1972) and U.S. Patent US-A-3,703,440 to Shigetaka (November 21, 1972) discuss the use of oligosaccharides as the main constituent for use as a starch syrup. U.S. Patent US-A-3,728,132 to Tsuyama, et al. (April 17, 1973) and U.S. Patent US-A-3,894,146 to Tsuyama (July 8, 1975), discuss the use of oligosaccharides as a low cariogenic sweetener.

U.S. Patent US-A-4,435,389 to Mutai, et al. (March 6, 1984) discusses an oligosaccharide composition for promoting the growth of Bifidobacteria in human intestines. The oligosaccharide composition has a general formula of Gal-(gal)n-Glc, wherein "Gal" denotes a galactose residue, "Glc" a glucose residue, and "n" an integer of one to four. Bifidobacteria is a bacteria living in the human intestines with known beneficial physiological affects.

U.S. Patent US-A-4,160,026 to Iwamatsu (July 3, 1979) describes antibiotic oligosaccharides termed SF-1130-x₁ and SF-1130-x₂ which are produced by the fermentation of Streptomyces myxogenes SF-1130. Toxicity against a number of microorganisms, including Salmonella, as tested by formation of inhibition zones from paper discs impregnated with the compounds was disclosed. These substances are described as active antibiotic substances against gram-negative bacteria.

U.S. Patent US-A-4,316,894 to Omoto, et al. (February 23, 1982) discloses a compound designated as SF-1130-x₃ having a disclosed utility as a drug for suppressing blood sugar elevations after ingesting starch and/or sugars and as a weak antibacterial compound. Although a chemical structure is not provided, antibacterial activity was demonstrated in E. coli. SF-1130-x₃ is described as an oligosaccharide and detailed chemical characterizations of the substance are provided. SF-1130-x₃ is produced by fermentation of Streptomyces bacteria.

Chemical Abstracts, Vol. 100, No. 17, 23/04/84, Abstract Number 137377f teaches that certain defined fructo-oligosaccharides are effective as Bifido growth stimulating substances. The Veterinary Record, Vol. 106, No. 3, 19/01/80, p. 61 teaches that hatched chicks can be protected from infection by food poisoning Salmonellas by administration of a suspension of caccal material from an adult bird. The investigators disclosed that adult caccal microflora consists of a complex mixture including Coliforms, faecal streptococci, clostridia, lactobacilli and non-sporing anaerobes.

In view of the above, a new method for inhibiting the growth of Salmonella is highly desirable. Such a method is useful for controlling or limiting the population of *Salmonella* in the intestines of food animals as a means for preventing human *Salmonella* infections.

### Summary of the Invention

According to a first aspect of the present invention there is provided the use of a fructo-oligosaccharide in the manufacture of a bacteriostatic composition for inhibiting the growth of Salmonella in a food animal, said fructo-oligosaccharide comprising a sucrose molecule having from 1-8 fructose residues attached thereto.

According to a second aspect of the present invention there is provided a non-therapeutic process of inhibiting the growth of *Salmonella* in a food animal which comprises feeding to the animal a *Salmonella* growth inhibitor, wherein the *Salmonella* growth inhibitor is a fructo-oligosaccharide comprising a sucrose molecule having from 1 to 8 fructose residues attached thereto.

According to a third aspect of the present invention there is provided a non-therapeutic process of inhibiting the growth of *Salmonella* in a food animal in the presence of microorganisms of genera other than *Salmonella*, which comprises feeding to the animal a compound that is fermented by the other microorganisms at a rate greater than the rate at which that compound is fermented by *Salmonella*, wherein the compound is a fructo-oligosaccharide comprising a sucrose molecule having from 1 to 8 fructose residues attached thereto.

Preferably, the composition is an animal feed into which the fructo-oligosaccharide is incorporated as a *Salmonella* inhibitor. Preferably the food animal is poultry. Preferably the fructo-oligosaccharide is a compound selected from 1-kestose, nystose, 1-fructofuranosyl-nystose and mixtures thereof.

The fructo-oligosaccharide is characterised as a sucrose molecule having from 1 to 8 fructose residues. This class of compound is exemplified by a product, Neosugar, which includes as its components 1-kestose, nystose, and 1-fructofuranosyl-nystose The fructo-oligosaccharide may be present in a feed composition which also has a nutritive component.

A particular embodiment of the invention includes feeding the compound to a food animal to inhibit the growth of *Salmonella* in the intestines of the animal. A further embodiment includes feeding the compound to an animal having intestinal *Salmonella* which cannot ferment and intestinal microflora, such as *Lactobacillus* or *Streptococcus*, which cannot ferment the fructo-oligosaccharides. In this method, *Salmonella* are competitively inhibited by the enhanced growth of other bacteria.

### Detailed Description

One aspect of the present invention involves a method for inhibiting the growth of *Salmonella*. A particular application of the invention is the inhibition of *Salmonella* in the intestines of animals for the prevention of infection of humans who later ingest food products from the animals. Generally, the effective composition in the method is a mixture of fructo-oligosaccharides which inhibits growth of species of *Salmonella*. It is believed that inhibition occurs due to the inability of *Salmonella* to ferment the effective composition. The effective composition and specific embodiments of the effective composition will be discussed in more detail below. However, for the present, all embodiments will be generally referred to as the "effective composition."

The method for inhibiting the growth of Salmonella includes contacting a population of Salmonella with the effective composition. It has been found that in the presence of the effective composition, with only minimal amounts of carbohydrate sources other than the effective composition available, Salmonella fermentation activity is limited. The fermentation which does occur is thought to be fermentation of small quantities of glucose or sucrose present in the medium. While not wishing to be bound by theory, it is believed that the lack of fermentation activity is due to the inability of Salmonella to break down components of the effective composition into smaller sugar units. Therefore, according to this theory, in an environment where the effective composition is present, growth of Salmonella is inhibited by reduced carbohydrate availability. If the carbohydrate source of the environment consists primarily of the effective composition, inhibition is very strong. If the environment has other carbohydrate sources which can be used by Salmonella, inhibition occurs, but at a lower level.

The embodiment of the effective composition discussed above appears to inhibit growth of Salmonella due to the inability of the organism to ferment the effective composition.

A particular embodiment of the present method involves introducing the effective composition to the intestinal tract of a food animal. A wide variety of microflora are present in the intestines of all animals. In the intestines of many animals from which humans derive food, populations of Salmonella are present without any deleterious effects to the animals. However, transmission of a sufficient number of Salmonella organisms to a human can cause serious illness. By introducing the effective composition to the intestines of a food animal, the balance of intestinal microflora is shifted away from Salmonella in favor of other species of microflora. In this manner, the likelihood of transmission of Salmonella organisms to humans from food animals is reduced because the initial Salmonella population is smaller.

The present method is particularly effective when microflora which are not pathogenic to humans and which can ferment the effective composition are present in the intestines of the food animal. In such animals, growth of Salmonella is competitively inhibited by the enhanced growth of other forms of microflora. For example, it has been found that the effective composition can be fermented by Lactobacillus and Streptococcus. These microorganisms are commonly found in many food animals and will not cause human illness. Poultry are known to have Salmonella, Lactobacillus and Streptococcus populations in their intestines. By introducing the effective composition to poultry, the growth of non-pathogenic microflora is enhanced and the population of Salmonella decreases. The overall balance of microflora in the intestines of the poultry will be shifted in favor of bacteria not harmful to humans, Lactobacillus and Streptococcus. The likelihood of human infection by Salmonella is thereby decreased because the source population of Salmonella is reduced.

The effective composition of the present invention includes fructo-oligosaccharides which cannot be fermented by Salmonella. "Fructo-oligosaccharides", as used herein, refers to a trisaccharide having one or more fructose residues. This class includes mixtures of oligosaccharide molecules comprised of sucrose having from 1 to 8 fructose residues. The fructose residues are preferably attached by a beta 2-1 bond. The class is exemplified by the fructo-oligosaccharides in the Neosugar® produced by Meiji Seika and as described in U.S. Patent US-A-4,681,771.

Neosugar® is a mixture including 1-kestose, nystose, and 1-fructofuranosyl-nystose. Neosugar, as used herein, is more particularly defined as having between about 20% by weight and about 40% by weight 1-kestose, between about 20% by weight and about 55% by weight nystose, and between about 5% by weight and about 15% by weight 1-fructofuranosyl-nystose. The remaining portion of a Neosugar® mixture can include between about 4% by weight and about 45% by weight of a mixture of glucose and sucrose. In one form, Neosugar® G, the composition is a 75% syrup having between about 40% by weight and about 50% by weight of a mixture of glucose and sucrose, between about 20% by weight and about 30% by weight 1-kestose, between about 20% by weight and about 30% by weight nystose, and between about 2% by weight and about 8% by weight 1-fructofuranosyl-nystose. In another form, Neosugar® P, the composition is either a 75% syrup or a powder having between about 2% by weight and about 6% by weight of a mixture of glucose and sucrose, between about 30% by weight and about 40% by weight 1-kestose, between about 45% by weight and about 55% by weight nystose, and between about 5% by weight and about 15% by weight 1-fructofuranosyl-nystose. The structures of 1-kestose, nystose, and 1-fructofuranosyl-nystose are provided below. 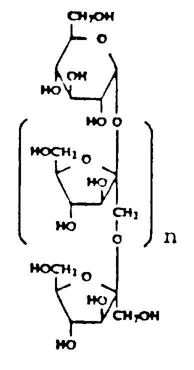
where:
n = 1 for 1-kestose
n = 2 for nystose
n = 3 for 1-fructofuranosylnystose
Neosugar® can be produced by the action of fructosyl-transferase on sucrose to produce a mixture of 1-kestose, nystose, and 1-fructofuranosyl nystose. Neosugar G, for example, can be produced by subjecting the product of fructosyl-transferase activity to decoloration, filtration, desalting, and concentration. Neosugar® G can be further purified with an ion exchange resin to produce Neosugar® P. Although these methods produce mixtures of fructo-oligosaccharides, it is contemplated that the use of the pure compounds which are in Neosugar® are within the scope of the invention.

Certain fungi, such as, Aspergillus and Aureobasidium are known to produce the enzyme fructosyltransferase. Fructosyl transferases which produce oligosaccharides are known to be produced by chicory plant and by onion plant. See Singh et al., Substrate Specificity of Fructosyl Transferase From Chicory Roots, Phytochemistry vol. 10, pp. 2037-39 (1971) and Henry et al., Sucrose: Sucrose Fructosyltransferase and Fructan:Fructan Fructosyltransferase From Allium Cepa, Phytochemistry vol. 19, pp. 1017-20 (1980).

In a preferred embodiment of the present method, the effective composition is fed to a food animal where the inhibition of the growth of intestinal Salmonella will occur. The preferred method of introduction is to mix the effective composition with nutritive feed material for the animal. It is contemplated, however, that the effective composition can either be mixed with the nutritive feed material or fed to the animal separately. In either embodiment, the effective composition must be provided in an amount effective to inhibit the growth of Salmonella. This amount will vary depending upon the size of the food animal. Poultry will require smaller quantities of the effective composition than, for example, beef to inhibit intestinal Salmonella. Effective amounts can readily be determined by experimentation.

In practice of the present method by feeding the effective composition to food animals to inhibit intestinal Salmonella populations, it is not necessary to practice the method for the entire life of the animal. The primary concern of the food industry is to prevent transmission of Salmonella to humans. Therefore, limiting the Salmonella population to a minimum by the present method just prior to slaughter of the food animal is sufficient to reduce the likelihood of transmission to the human population. In this manner, costs attendant to the present process can be minimized.

The feed composition used in the present invention includes, as one component, the effective composition. The feed composition also includes some material which is nutritive for the animal to which the feed composition is fed. Typically, for most food animals, such as poultry or beef, the nutritive material is some type of grain product. It is contemplated that the majority of the feed composition can be nutritive material with the effective composition present in an amount sufficient to inhibit growth of intestinal Salmonella. Typically, the effective composition is present in an amount between about 0.05% by weight and about 5% by weight and more preferably between about 0.25% by weight and about 3% by weight and most preferably between about 0.25% by weight and about 1% by weight.

The method of the present invention can be used for inhibiting the growth of Salmonella in a wide variety of animals from which humans obtain food. Many such animals are known to have intestinal Salmonella populations, and therefore, can potentially contaminate any meat or dairy products consumed by humans. Accordingly, the present method is contemplated for use with any type of food animal, including but not limited to, poultry, beef, pork, and lamb. The term "poultry" is meant to include, but not be limited to, chickens, ducks, turkeys, geese, quail, and rock cornish game hens.

Another aspect of the present invention is a method for reducing or preventing the intestinal colonization of food animals by Salmonella which includes introducing the effective composition to the intestinal tract of a food animal and also introducing a competitive exclusion culture to the intestinal tract of the food animal. As used herein, the term "reducing" shall refer to the concept of preventing as well. The term "competitive exclusion" refers to a recognized method of preventing the intestinal colonization of young food animals by pathogenic bacteria, such as Salmonella. This method is discussed, for example, in Stavric, Microbial Colonization Control of Chicken Intestine Using Defined Cultures, Food Technology 41(7), pp. 93-98, July 1987.

Competitive exclusion involves the introduction of a culture of normal adult intestinal microflora into the intestines of a young animal to protect against the colonization of the intestines of the young animal by undesirable microorganisms, such as Salmonella. Typically, when animals are born, few genera of microorganisms are present in the gut. Native adult microflora, however, become established in poultry, for example, in the small intestine within about two weeks and in the ceca within about four weeks. In modern breeding methods food animals are often reared in the absence of adult animals and the normal, healthy gut microflora which would naturally be transferred from adults to the young are absent in the environment of the young animals. Animals raised under such conditions are particularly susceptible to gut colonization by pathogenic bacteria, such as Salmonella. By providing competitive exclusion cultures to newborn animals, the establishment of normal adult microflora to the exclusion of Salmonella or other harmful bacteria is facilitated.

Introduction of the competitive exclusion culture into the young animals should occur early in the life of the animal so that the introduced culture has time to become established prior to challenge by an unwanted culture of pathogenic bacteria. For example, as discussed in Starvic, it has been found that chicks can be successfully made resistant to Salmonella infection by innoculating one to two day old chicks with an undefined culture from healthy adult Salmonella-free chickens.

Competitive exclusion cultures are either "defined" or "undefined". Undefined cultures refer to microflora cultures which are taken from the intestines of healthy adult animals having established intestinal microflora and which are not infected by Salmonella. For example, it has been found that chickens reared under normal conditions develop fully protective intestinal microflora cultures within about three to five weeks. Defined competitive exclusion cultures refer to bacterial cultures in which the genera of bacteria in the culture are known. Defined cultures of a single species of bacteria have been experimented with, as well as defined cultures including numerous species of bacteria. Competitive exclusion cultures whether defined or undefined are introduced orally to the treated animal.

By using competitive exclusion techniques in conjunction with the introduction of the effective composition to young food animals, intestinal colonization of Salmonella is reduced. As discussed above, competitive exclusion cultures are preferably provided early in the life of an animal, and in the instance of chickens, are preferably provided within the first two days of life. As discussed above, the present method of feeding the effective composition to food animals to inhibit intestinal Salmonella populations, can be conducted either throughout the entire life of the food animal or only during a portion thereof. For example, if administration of a competitive exclusion culture is used, feeding of the effective composition can be conducted during the first several days or several weeks of life around the time the competitive exclusion culture is being administered. Alternatively, the effective composition can be fed to the food animal throughout its entire life while the competitive exclusion culture is administered as discussed above, i.e., within the early part of the life of the animal. Another alternative is to feed the effective composition to the food animal either just prior to slaughter of the food animal or, for example, during the initial several days or several weeks of life while the competitive exclusion culture is being administered and during the time period just prior to slaughter of the food animal.

Another aspect of the present invention includes the use of feeding the effective composition to food animals in a "shuttle program" with antibiotics to reduce the colonization or growth of Salmonella populations in the food animals' intestinal tract. Such a shuttle program involves the alternating use of the effective composition and one or more orally administered antibiotics. The antibiotics used in a shuttle program can include any antibiotic which is recognized as being effective against Salmonella or which hereafter becomes recognized as being effective against Salmonella. Such antibiotics include, without limitation, chloramphenicol, ampicillin, trimethoprim-sulfa, and mixtures thereof.

The effective composition and any antibiotics used can be administered according to various time schedules. For example, the effective composition and the antibiotic[s] can be administered simultaneously or serially. The required frequency of administrations depends upon the conditions to which the food animals are exposed. Under conditions in which the food animals are often exposed to strong Salmonella challenges and other environmental stress, such as extreme heat or cold or overcrowded conditions, the frequency of administrations should be greater. The frequency of administration of a shuttle program using the effective composition and antibiotics can be the same as that for typical antibiotic administration programs. The administration of the effective composition and antibiotics can be provided serially with time periods between administrations varying from several minutes to several hours to several days, and preferably by at least about one day. In addition, a shuttle program can involve the use of the effective composition on a regular basis in the feed supply in conjunction with periodic administrations of antibiotics according to well known methods of antibiotic use.

The amount of the effective composition used in administrations in a shuttle program will typically be the same as discussed above for other uses of the effective compound. The amount of antibiotics used in a shuttle program will typically be the same as other uses of antibiotics in animal breeding and will vary between antibiotics.

In a shuttle program, the effective compound and antibiotics can be used in either a preventative or curative manner. If used in a preventative manner, the shuttle program should be initiated shortly after birth of the food animal to prevent the initial colonization of the intestinal track with pathogenic bacteria. Such preventative use should preferably start within about two days of birth. However, it should be recognized that when used in a preventative manner, a shuttle program can be initiated at any time to food animals that are not colonized by pathogenic bacteria.

A shuttle program can also be used in a curative manner. When a food animal becomes infected with pathogenic bacteria, the use of a shuttle program can effectively slow the growth of, reduce, or eliminate the infection.

The following experimental results are provided for purposes of illustration and are not intended to limit the scope of the invention. The bacterial strains in the following examples were obtained from Colorado Animal Research Enterprises, Inc., 6200 E. County Road 56, Ft. Collins, Colorado 80524.

### EXPERIMENTAL

### Example 1

A strain of Salmonella typhimurium, species source poultry, obtained from Lilly, No. 289-1 was tested for the ability to metabolize Neosugar P. This ability was measured by acid production as measured by Phenol Red Broth Base (PRB). Growth, as measured by turbidity, was also tested.

A fermentation medium of PRB (Difco) was prepared and sterilized according to manufacturer's instructions. PRB is a defined medium which lacks a carbohydrate source. If an added carbohydrate source, such as Neosugar P, is fermented, the medium turns yellow as a positive response due to acid formed by the fermentation. 5.0 ml of the PRB was dispended into 10 test tubes. A 70% solution of Neosugar P was diluted by 1:7 in deionized water and filter sterilized. 0.5 ml of the diluted, sterile Neosugar p solution was aseptically added to individual PRB tubes to provide a 1.0% concentration of sugar in each tube. Five of the ten tubes were overlaid with mineral oil to simulate anaerobic conditions. After incubation at 37°C ± 1°C, acid production and growth were measured at 24, 48, and 72 hours. The strain was also tested for viability on Tryptic-Soy (T-Soy) Agar plates and tested for presence of Salmonella on Salmonella-Shigella (SS) Agar plates. The results of these tests are provided in Table 1.

The symbol "w+" means "weak positive", "+" means "positive", "++" means "strong positive", "+++" means "strongest positive", and "-" means "no detectable positive response".

**TABLE 1**

| S. typhimurium | | | | | | |
|---|---|---|---|---|---|---|
| Test Tube | Acid Formation | | | Growth | | |
| | 24 | 48 | 72 | 24 | 48 | 72 |
| 1 | w+ | - | - | + | + | ++ |
| 2 | w+ | - | - | + | + | ++ |
| 3 | w+ | - | - | + | + | ++ |
| 4 | w+ | - | - | + | + | ++ |
| 5 | w+ | - | - | + | + | ++ |
| 6* | w+ | - | - | + | + | ++ |
| 7* | w+ | - | - | + | + | ++ |
| 8* | w+ | - | - | + | + | ++ |
| 9* | w+ | - | - | + | + | ++ |
| 10* | w+ | - | - | + | + | ++ |
| SS Agar - very good growth; yellow (ferments lactose); some black colonies T-Soy Agar - very good growth | | | | | | |

| | | | | | | |
|---|---|---|---|---|---|---|
| * - anaerobic | | | | | | |

### Example 2

A strain of Salmonella typhimurium from cattle, FDA No. 2952 was tested according to the procedure in Example 1. The results of these tests are provided in Table 2.

**TABLE 2**

| S. typhimurium | | | | | | |
|---|---|---|---|---|---|---|
| Test Tube | Acid Formation | | | Growth | | |
| | 24 | 48 | 72 | 24 | 48 | 72 |
| 1 | w+ | - | - | + | + | ++ |
| 2 | w+ | - | - | + | + | ++ |
| 3 | w+ | - | - | + | + | ++ |
| 4 | w+ | - | - | + | + | ++ |
| 5 | w+ | - | - | + | + | ++ |
| 6* | w+ | - | - | + | + | ++ |
| 7* | w+ | - | - | + | + | ++ |
| 8* | w+ | - | - | + | + | ++ |
| 9* | w+ | - | - | + | + | ++ |
| 10* | w+ | - | - | + | + | ++ |
| SS Agar - very good growth; yellow (ferments lactose); some black colonies T-Soy Agar - very good growth | | | | | | |

| | | | | | | |
|---|---|---|---|---|---|---|
| * - anaerobic | | | | | | |

### Example 3

A strain of Salmonella typhimurium, from cattle, NVSL No. 82-4481, was tested according to the procedure in Example 1. The results of these tests are provided in Table 3.

**TABLE 3**

| S. typhimurium | | | | | | |
|---|---|---|---|---|---|---|
| Test Tube | Acid Formation | | | Growth | | |
| | 24 | 48 | 72 | 24 | 48 | 72 |
| 1 | w+ | - | - | + | + | ++ |
| 2 | w+ | - | - | + | + | ++ |
| 3 | w+ | - | - | + | + | ++ |
| 4 | w+ | - | - | + | + | ++ |
| 5 | w+ | - | - | + | + | ++ |
| 6* | w+ | w+ | - | + | + | ++ |
| 7* | w+ | - | - | + | + | ++ |
| 8* | w+ | - | - | + | + | ++ |
| 9* | w+ | - | - | + | + | ++ |
| 10* | w+ | - | - | + | + | ++ |
| SS Agar - good growth; yellow; black colonies T-Soy Agar - very good growth | | | | | | |

| | | | | | | |
|---|---|---|---|---|---|---|
| * - anaerobic | | | | | | |

### Example 4

A strain of Salmonella typhimurium from swine, NVSL No. 83-31641-4807 was tested according to the procedure in Example 1. The results of these tests are provided in Table 4.

**TABLE 4**

| S. typhimurium | | | | | | |
|---|---|---|---|---|---|---|
| Test Tube | Acid Formation | | | Growth | | |
| | 24 | 48 | 72 | 24 | 48 | 72 |
| 1 | w+ | - | - | + | + | ++ |
| 2 | w+ | - | - | + | + | ++ |
| 3 | w+ | - | - | + | + | ++ |
| 4 | w+ | - | - | + | + | ++ |
| 5 | w+ | - | - | + | + | ++ |
| 6* | w+ | w+ | - | + | + | ++ |
| 7* | w+ | w+ | - | + | + | ++ |
| 8* | w+ | - | - | + | + | ++ |
| 9* | w+ | - | - | + | + | ++ |
| 10* | w+ | - | - | + | + | ++ |
| SS Agar - good growth; yellow; black colonies T-Soy Agar - good growth | | | | | | |

| | | | | | | |
|---|---|---|---|---|---|---|
| * - anaerobic | | | | | | |

### Example 5

A strain of Salmonella typhimurium from swine, NVSL No. 83-31296-4756 was tested according to the procedure in Example 1. The results of these tests are provided in Table 5.

**TABLE 5**

| S. typhimurium | | | | | | |
|---|---|---|---|---|---|---|
| Test Tube | Acid Formation | | | Growth | | |
| | 24 | 48 | 72 | 24 | 48 | 72 |
| 1 | w+ | - | - | + | + | ++ |
| 2 | - | w+ | - | + | + | ++ |
| 3 | w+ | - | - | + | + | ++ |
| 4 | w+ | - | - | + | + | ++ |
| 5 | - | - | - | + | + | ++ |
| 6* | w+ | w+ | - | + | + | ++ |
| 7* | w+ | w+ | - | + | + | ++ |
| 8* | w+ | - | - | + | + | ++ |
| 9* | w+ | - | - | + | + | ++ |
| 10* | w+ | - | - | + | + | ++ |
| SS Agar - good growth; yellow; some black T-Soy Agar - good growth | | | | | | |

| | | | | | | |
|---|---|---|---|---|---|---|
| * - anaerobic | | | | | | |

### Example 6

A strain of Escherichia coli from poultry, Pfizer No. BO28, was tested according to the procedure in Example 1. The results of these tests are provided in Table 6.

**TABLE 6**

| E. coli | | | | | | |
|---|---|---|---|---|---|---|
| Test Tube | Acid Formation | | | Growth | | |
| | 24 | 48 | 72 | 24 | 48 | 72 |
| 1 | - | - | - | + | + | ++ |
| 2 | - | - | - | + | + | ++ |
| 3 | w+ | - | - | + | + | ++ |
| 4 | w+ | - | - | + | + | ++ |
| 5 | w+ | - | - | + | + | ++ |
| 6* | + | w+ | - | + | + | ++ |
| 7* | + | w+ | - | + | + | ++ |
| 8* | + | w+ | - | + | + | ++ |
| 9* | + | w+ | - | + | + | ++ |
| 10* | + | w+ | - | + | + | ++ |
| SS Agar - single colony; black red T-Soy Agar - good growth; 2-3 mm | | | | | | |

| | | | | | | |
|---|---|---|---|---|---|---|
| * - anaerobic | | | | | | |

### Example 7

A strain of Escherichia coli from poultry, NVSL No. 80-430 was tested according to the procedure in Example 1. The results of these tests are provided in Table 7.

**TABLE 7**

| E. coli | | | | | | |
|---|---|---|---|---|---|---|
| Test Tube | Acid Formation | | | Growth | | |
| | 24 | 48 | 72 | 24 | 48 | 72 |
| 1 | w+ | - | - | + | + | ++ |
| 2 | w+ | - | - | + | + | ++ |
| 3 | w+ | - | - | + | + | ++ |
| 4 | w+ | - | - | + | + | ++ |
| 5 | w+ | - | - | + | + | ++ |
| 6* | ++ | + | w+ | + | + | ++ |
| 7* | ++ | + | w+ | + | + | ++ |
| 8* | ++ | + | w+ | + | + | ++ |
| 9* | ++ | + | w+ | + | + | ++ |
| 10* | ++ | + | w+ | + | + | ++ |
| SS Agar - no growth T-Soy Agar - good growth; 2mm; motile | | | | | | |

| | | | | | | |
|---|---|---|---|---|---|---|
| * - anaerobic | | | | | | |

### Example 8

A strain of Escherichia coli from cattle, NVSL No. 85-688 was tested according to the procedure in Example 1. The results of these tests are provided in Table 8.

**TABLE 8**

| E. coli | | | | | | |
|---|---|---|---|---|---|---|
| Test Tube | Acid Formation | | | Growth | | |
| | 24 | 48 | 72 | 24 | 48 | 72 |
| 1 | w+ | - | - | + | + | +++ |
| 2 | w+ | - | - | + | + | +++ |
| 3 | w+ | - | - | + | + | +++ |
| 4 | w+ | - | - | + | + | +++ |
| 5 | w+ | - | - | + | + | +++ |
| 6* | + | + | w+ | + | + | +++ |
| 7* | + | + | w+ | + | + | +++ |
| 8* | + | + | w+ | + | + | +++ |
| 9* | + | + | w+ | + | + | +++ |
| 10* | + | + | w+ | + | + | +++ |
| SS Agar - single colony; black red T-Soy Agar - good growth; 2-3 mm | | | | | | |

| | | | | | | |
|---|---|---|---|---|---|---|
| * - anaerobic | | | | | | |

### Example 9

A strain of Escherichia coli from swine, University of Guelp, G491 was tested according to the procedure in Example 1. The results of these tests are provided in Table 9.

**TABLE 9**

| E. coli | | | | | | |
|---|---|---|---|---|---|---|
| Test Tube | Acid Formation | | | Growth | | |
| | 24 | 48 | 72 | 24 | 48 | 72 |
| 1 | w+ | w+ | +++ | + | + | +++ |
| 2 | w+ | w+ | +++ | + | + | +++ |
| 3 | w+ | w+ | +++ | + | + | +++ |
| 4 | w+ | w+ | +++ | + | + | +++ |
| 5 | w+ | w+ | +++ | + | + | +++ |
| 6* | ++ | ++ | +++ | + | + | +++ |
| 7* | ++ | ++ | +++ | + | + | +++ |
| 8* | ++ | ++ | +++ | + | + | +++ |
| 9* | ++ | ++ | +++ | + | + | +++ |
| 10* | ++ | ++ | +++ | + | + | +++ |
| SS Agar - selected colonies; pink/red T-Soy Agar - good growth; 2-3 mm | | | | | | |

| | | | | | | |
|---|---|---|---|---|---|---|
| * - anaerobic | | | | | | |

### Example 10 A strain of Escherichia coli from swine, NVSL No. 85-746 was tested according to the procedure in Example 1. The results of these tests are provided in Table 10.

**TABLE 10**

| E. coli | | | | | | |
|---|---|---|---|---|---|---|
| Test Tube | Acid Formation | | | Growth | | |
| | 24 | 48 | 72 | 24 | 48 | 72 |
| 1 | ++ | ++ | +++ | ++ | ++ | +++ |
| 2 | ++ | ++ | +++ | ++ | ++ | +++ |
| 3 | ++ | ++ | +++ | ++ | ++ | +++ |
| 4 | ++ | ++ | +++ | ++ | ++ | +++ |
| 5 | ++ | ++ | +++ | ++ | ++ | +++ |
| 6* | +++ | +++ | +++ | ++ | ++ | +++ |
| 7* | +++ | +++ | +++ | ++ | ++ | +++ |
| 8* | +++ | +++ | +++ | ++ | ++ | +++ |
| 9* | +++ | +++ | +++ | ++ | ++ | +++ |
| 10* | +++ | +++ | +++ | ++ | ++ | +++ |
| SS Agar - no growth T-Soy Agar - very good growth; 3 mm; motile | | | | | | |

| | | | | | | |
|---|---|---|---|---|---|---|
| * - anaerobic | | | | | | |

### Example 11

A strain of Streptococcus faecalis obtained from the Center for Disease Control, STR-11 was tested according to the procedure in Example 1. The results of these tests are provided in Table 11.

**TABLE 11**

| Streptococcus faecalis | | | | | | |
|---|---|---|---|---|---|---|
| Test Tube | Acid Formation | | | Growth | | |
| | 24 | 48 | 72 | 24 | 48 | 72 |
| 1 | ++ | +++ | +++ | + | ++ | +++ |
| 2 | ++ | +++ | +++ | + | ++ | +++ |
| 3 | ++ | +++ | +++ | + | ++ | +++ |
| 4 | ++ | +++ | +++ | + | ++ | +++ |
| 5 | ++ | +++ | +++ | + | ++ | +++ |
| 6* | ++ | +++ | +++ | + | ++ | +++ |
| 7* | ++ | +++ | +++ | + | ++ | +++ |
| 8* | ++ | +++ | +++ | + | ++ | +++ |
| 9* | ++ | +++ | +++ | + | ++ | +++ |
| 10* | ++ | +++ | +++ | + | ++ | +++ |
| SS Agar - no growth T-Soy Agar - small white colonies | | | | | | |

| | | | | | | |
|---|---|---|---|---|---|---|
| * - anaerobic | | | | | | |

### Example 12

A strain of Streptococcus faecalis obtained from Colorado State University Microbiology Department Culture Collection was tested according to the procedure in Example 1. The results of these tests are provided in Table 12.

**TABLE 12**

| Streptococcus faecalis | | | | | | |
|---|---|---|---|---|---|---|
| Test Tube | Acid Formation | | | Growth | | |
| | 24 | 48 | 72 | 24 | 48 | 72 |
| 1 | ++ | +++ | +++ | + | ++ | +++ |
| 2 | ++ | +++ | +++ | + | ++ | +++ |
| 3 | ++ | +++ | +++ | + | ++ | +++ |
| 4 | ++ | +++ | +++ | + | ++ | +++ |
| 5 | ++ | +++ | +++ | + | ++ | +++ |
| 6* | ++ | +++ | +++ | + | ++ | +++ |
| 7* | ++ | +++ | +++ | + | ++ | +++ |
| 8* | ++ | +++ | +++ | + | ++ | +++ |
| 9* | ++ | +++ | +++ | + | ++ | +++ |
| 10* | ++ | +++ | +++ | + | ++ | +++ |
| SS Agar - no growth T-Soy Agar - small white colonies; 1 mm | | | | | | |

| | | | | | | |
|---|---|---|---|---|---|---|
| * - anaerobic | | | | | | |

### Example 13

A strain of Lactobacillus plantarum, obtained from Colorado State University Microbiology Department Culture Collection was tested according to the procedure in Example 1. The results of these tests are provided in Table 13.

**TABLE 13**

| Lactobacillus plantarum | | | | | | |
|---|---|---|---|---|---|---|
| Test Tube | Acid Formation | | | Growth | | |
| | 24 | 48 | 72 | 24 | 48 | 72 |
| 1 | + | ++ | +++ | w+ | + | ++ |
| 2 | + | ++ | +++ | w+ | + | ++ |
| 3 | + | ++ | +++ | w+ | + | ++ |
| 4 | + | ++ | +++ | w+ | + | ++ |
| 5 | - | ++ | +++ | - | + | ++ |
| 6* | + | ++ | +++ | w+ | + | ++ |
| 7* | + | ++ | +++ | w+ | + | ++ |
| 8* | + | ++ | +++ | w+ | + | ++ |
| 9* | + | ++ | +++ | w+ | + | ++ |
| 10* | + | ++ | +++ | w+ | + | ++ |
| SS Agar - no growth T-Soy Agar - poor growth; very small colonies | | | | | | |

| | | | | | | |
|---|---|---|---|---|---|---|
| * - anaerobic | | | | | | |

From the above experiments, it can be seen that the Salmonella strains exhibited little fermentation activity as measured by acid formation. While all strains showed some initial "weak positive" results, this initial activity likely indicates fermentation of the glucose and sucrose in the Neosugar composition.

All of the Salmonella strains showed consistent moderate growth as measured by turbidity. The lack of fermentation while growth occurred indicates that the Neosugar was not the carbon source for any growth of Salmonella. It appears, therefore, that some other carbon source, such as, for example, amino acids, in the PRB was used by Salmonella. The fact that Salmonella is able to grow in the presence of Neosugar indicates that, although the organism is unable to ferment the fructo-oligosaccharides in Neosugar, these compositions are not toxic to Salmonella at the concentrations in these tests.

## Claims

1. The use of a fructo-oligosaccharide in the manufacture of a bacteriostatic composition for inhibiting the growth of Salmonella in a food animal, said fructo-oligosaccharide comprising a sucrose molecule having from 1-8 fructose residues attached thereto.

2. The use according to claim 1 wherein the composition is an animal feed into which the fructo-oligosaccharide is incorporated as a *Salmonella* inhibitor.

3. The use according to claim 1 or claim 2 wherein the food animal is poultry.

4. The use according to any one of the preceding claims wherein the fructo-oligosaccharide is a compound selected from 1-kestose, nystose, 1-fructofuranosyl-nystose and mixtures thereof.

5. A non-therapeutic process of inhibiting the growth of *Salmonella* in a food animal which comprises feeding to the animal a *Salmonella* growth inhibitor, wherein the *Salmonella* growth inhibitor is a fructo-oligosaccharide comprising a sucrose molecule having from 1 to 8 fructose residues attached thereto.

6. A non-therapeutic process of inhibiting the growth of *Salmonella* in a food animal in the presence of microorganisms of genera other than *Salmonella*, which comprises feeding to the animal a compound that is fermented by the other microorganisms at a rate greater than the rate at which that compound is fermented by *Salmonella*, wherein the compound is a fructo-oligosaccharide comprising a sucrose molecule having from 1 to 8 fructose residues attached thereto.

7. A process according to claim 5 or claim 6 wherein the fructo-oligosaccharide is a compound selected from 1-kestose, nystose, 1-fructofuranosyl-nystose and mixtures thereof.

8. A process according to any one of claims 5 to 7 wherein the *Salmonella* growth inhibitor is fed to the animal in combination with a competitive exclusion culture.

9. A process according to any one of claims 5 to 8 wherein the food animal is poultry.

## Patentansprüche

1. Die Verwendung von einem Fructo-Oligosaccharid zur Herstellung einer bakteriostatischen Zusammensetzung zur Inhibierung des Wachstums von Salmonellen in einem Nutztier, wobei die Fructo-Oligosaccharide ein Saccharosemolekül aufweisen, an das 1-8 Fructosereste gebunden sind.

2. Verwendung gemäß Anspruch 1, wobei die Zusammensetzung ein Tierfutter ist, in das die Fructo-Oligosaccharide als Salmellen-Inhibitor eingebracht sind.

3. Die Verwendung gemäß Anspruch 1 oder Anspruch 2, wobei das Nutztier Geflügel ist.

4. Die Verwendung gemäß irgendeinem der vorstehenden Ansprüche, wobei das Fructo-Oligosaccharid eine Verbindung ausgewählt aus 1-Kestose, Nystose, 1-Fructofuranosyl-Nystose und Mischungen davon ist.

5. Ein nicht-therapeutisches Verfahren zur Inhibierung des Wachstums von Samonellen in einem Nutztier, umfassend das Füttern an das Tier als Samonellen-Wachstumsinhibitor, worin der Salmonellen-Wachstuminhibitor ein Fructo-Oligosaccharid mit einem Saccharosemolekül ist, an das 1 bis 8 Fructosereste gebunden sind.

6. Ein nicht-therapeutisches Verfahren zur Inbibierung des Wachstums von Salmonellen in einem Nutztier in Anwesenheit von Mikroorganismen von anderer Art als Salmonellen, umfassend das Füttern einer Zusammensetzung an ein Tier, die durch die anderen Mikroorganismen mit einer größeren Rate fermentiert wird, als die Rate, bei der diese Mischung von Salmonellen fermentiert wird, worin die Zusammensetzung ein Fructo-Oligosaccharid ist, welches ein Sacccharosemolekül aufweist, an das 1 bis 8 Fructosereste gebunden sind.

7. Ein Verfahren gemäß Anspruch 5 oder Anspruch 6, wobei das Fructo-Oligosaccharid eine Verbindung ausgewählt aus 1-Kestose, Nystose, 1-Fructofuranosyl-Nystose und Mischungen davon ist.

8. Ein Verfahren gemäß irgendeinem der Ansprüche 5 bis 7, wobei der Salmonella-Wachstumsinhibitor an das Tier in Verbindung mit einer konkurrierenden Ausschlußkultur gefüttert wird.

9. Ein Verfahren gemäß irgendeinem der Ansprüche 5 bis 8, wobei das Nutztier Geflügel ist.

## Revendications

1. Utilisation d'un fructo-oligosaccharide dans la fabrication d'une composition bactériostatique pour inhiber la croissance des salmonelles chez un animal destiné à l'alimentation humaine, ledit fructo-oligosaccharide comprenant une molécule de saccharose ayant de 1 à 8 groupements fructose reliés entre eux.

2. Utilisation selon la revendication 1, caractérisée en ce que la composition est un aliment du bétail dans lequel le fructo-oligosaccharide est incorporé comme inhibiteur des salmonelles.

3. Utilisation selon la revendication 1 ou 2, caractérisée en ce que l'animal destiné à l'alimentation humaine est une volaille.

4. Utilisation selon l'une quelconque des revendications précédentes, caractérisée en ce que le fructo-oligosaccharide est un composé sélectionné parmi le 1-kestose, le nystose, le 1-fructofuranosyl-nystose et leurs mélanges.

5. Procédé non thérapeutique pour inhiber la croissance des salmonelles chez un animal destiné à l'alimentation humaine, caractérisé en ce qu'il consiste à donner comme aliment, à un animal, un inhibiteur de croissance des salmonelles, où ledit inhibiteur de croissance des salmonelles est un fructo-oligosaccharide comprenant une molécule de saccharose ayant de 1 à 8 groupements fructose reliés entre eux.

6. Procédé non thérapeutique pour inhiber la croissance des salmonelles chez un animal destiné à l'alimentation humaine, en présence de micro-organismes d'espèces différentes des salmonelles, caractérisé en ce qu'il consiste à donner comme aliment, à l'animal, un composé qui est fermenté par les autres micro-organismes à une vitesse supérieure à la vitesse à laquelle ce composé est fermenté par les salmonelles, où ledit composé est un fructo-oligosaccharide comprenant une molécule de saccharose ayant de 1 à 8 groupements fructose reliés entre eux.

7. Procédé selon la revendication 5 ou 6, caractérisé en ce que le fructo-oligosaccharide est un composé sélectionné parmi le 1-kestose, le nystose, le 1-fructofuranosyl-nystose et leurs mélanges.

8. Procédé selon l'une quelconque des revendications 5 à 7, caractérisé en ce que l'inhibiteur de croissance des salmonelles est administré à l'animal en combinaison avec une culture compétitive d'exclusion.

9. Procédé selon l'une quelconque des revendications 5 à 8, caractérisé en ce que l'animal destiné à l'alimentation humaine est une volaille.
